# EUROPÄISCHE PATENTSCHRIFT

(11) **EP 2 594 302 B1**
(45) Veröffentlichungstag und Bekanntmachung des Hinweises auf die Patenterteilung: **07.06.2017**
(21) Anmeldenummer: 12180587.3
(22) Anmeldetag: 15.08.2012
(51) Int. Cl.: A61M 15/08, A61L 9/04, B05B 11/00, B65D 47/00

(54) **Nasalspender**
Nasal dispenser
Distributeur nasal

(30) Priorität: 18.11.2011 DE 102011086677
(43) Veröffentlichungstag der Anmeldung: 22.05.2013
(73) Patentinhaber: Aptar Radolfzell GmbH, 78315 Radolfzell (DE)
(72) Erfinder: Dams, Ralf J., 78343 Gaienhofen (DE)
(74) Vertreter: Patentanwaltskanzlei Cartagena

(56) Entgegenhaltungen:
- WO-A1-01/23024
- DE-A1- 19 736 999
- DE-A1- 19 739 989
- US-A- 2 987 261
- US-A- 5 109 839
- US-A1- 2003 127 102
- Ansgar Behler: "Geruchsverbesserungsmittel", ROEMPP LEXIKON CHEMIE , November 2005 (2005-11), XP002691272, Gefunden im Internet: URL:http://www.roempp.com/prod/roempp.php [gefunden am 2013-01-31]

## Beschreibung

### Anwendungsgebiet und Stand der Technik

Die Erfindung betrifft einen Spender zum Austrag einer pharmazeutischen Flüssigkeit auf nasalem Wege. Ein solcher gattungsgemäßer Spender umfasst ein Flüssigkeitsreservoir und eine Nasenolive zur Einführung in ein Nasenloch des Benutzers. Dabei ist das Flüssigkeitsreservoir durch einen Förderkanal mit einer an der Nasenolive angeordneten Auslassöffnung verbunden, so dass durch Betätigung des Spenders ein Austragvorgang bewirkt werden kann.

Gattungsgemäße Spender sind aus dem Stand der Technik allgemein bekannt. Es gibt sie in verschiedenen Ausgestaltungen. Bei sehr einfachen Spendern ist vorgesehen, dass das Flüssigkeitsreservoir als Ganzes kraftbeaufschlagt wird, um Flüssigkeit durch die Nasenolive hindurch nach außen zu fördern. Üblicher sind Ausgestaltungen, bei denen im Förderkanal eine Pumpeinrichtung vorgesehen ist, welche mittels einer separaten Betätigungshandhabe betätigt werden kann, um Flüssigkeit aus dem Flüssigkeitsreservoir zur Nasenolive und zur Austragöffnung zu fördern.

Bei gattungsgemäßen Spendern ist vorgesehen, dass die Nasenolive zumindest zum Teil in das Nasenloch des Benutzers eingeführt wird. Sie weist eine hierfür geeignete sich konisch zur Austragöffnung hin verjüngende Formgebung auf. Bei einem solchen Spender mit Nasenolive ist es unvermeidbar, dass der Benutzer den Eigengeruch des Spenders wahrnehmen kann. Auch ist bei nasal zugeführten pharmazeutischen Flüssigkeiten unvermeidbar, dass auch deren Geruch für den Benutzer erfassbar ist.

Sowohl der Spender selbst als auch die auszutragende Flüssigkeit können jedoch einen unangenehmen Geruch haben.

In Hinblick auf die pharmazeutischen Flüssigkeiten, deren Austrag mit einem gattungsgemäßen Spender vorgesehen ist, ist es bereits bekannt, durch eine Aromatisierung der Flüssigkeit dieser ihren mitunter unangenehmen Geruch zu nehmen bzw. diesen zu überdecken. Durch eine solche Aromatisierung kann auch der möglicherweise unangenehm empfundene Geruch des Spenders gemildert werden. Dieser unangenehme Geruch des Spenders selbst kann durch den Kunststoff selbst verursacht sein. Es wurde festgestellt, dass auch die im Zusammenhang mit konservierungsmittelfreien Flüssigkeiten übliche Bestrahlung des Spenders zum Zwecke der Keimtötung, insbesondere die Bestrahlung mit Gammastrahlen, einen unangenehmen Geruch am Spender hinterlassen kann.

Aus dem Stand der Technik ist es weiterhin bekannt, Kunststoffe bereits während der Herstellung eines daraus gefertigten Produktes um Aromastoffe zu ergänzen. So offenbart die DE 36 058 81 A1 derartiges im Zusammenhang mit Kunststofftüten, die aus Kunststofffolie hergestellt sind. US2003/0127102 und US5109839 beschreiben aromatisierten Kunststoffmasken die mit Gasen in Kontakt treten.

### Geänderte Beschreibungsseiten 3 bis 6

### Aufgabe und Lösung

Aufgabe der Erfindung ist es, einen gattungsgemäßen Spender dahingehend weiterzubilden, dass dieser die oben skizzierten Nachteile des Standes der Technik vermindert.

Erfindungsgemäß wird dies dadurch erzielt, dass mindestens ein Teil des Spenders aus einem aromatisierten Kunststoff besteht, wobei die Aromatisierung mit einem Aroma aus der Gruppe umfassend Anisaroma, Campheraroma, Salbeiaroma, Minzaroma, insbesondere Pfefferminzaroma, Kamillearoma, Mentholaroma, Citronellaroma, Eukalyptusaroma, Fichtennadelaroma, Lavendelaroma, Nelkenaroma, Rosenaroma, Honigaroma und Fruchtaroma, insbesondere Beerenaroma, bevorzugt Citrusaroma, erfolgt. Auch Mischungen mit mehreren Aromen können im Einzelfall zweckmäßig sein.

In anderen Worten ist der aromatisierte Kunststoff in vorteilhaften Ausführungsformen mit einem oder mehreren aus Anis, Campher, Salbei, Minze, insbesondere Pfefferminze, Kamille, Menthol, Citronell, Eukalyptus, Fichtennadeln, Lavendel, Nelken, Rosen, Honig und/oder Früchten, insbesondere Beerenfrüchten, bevorzugt Citrusfrüchten, stammenden Aromastoffen versehen.

Erfindungsgemäß ist somit vorgesehen, dass der Spender selbst mit einem Eigengeruch versehen wird, indem zumindest ein aus Kunststoff bestehendes Bauteil als Bauteil aus einem aromatisierten Kunststoff ausgebildet ist. Unter einem aromatisierten Kunststoff ist im Sinne der Erfindung ein Kunststoff zu verstehen, der mit einem oder mehreren Aromastoffen versehen ist. Der bzw. die zugesetzten Aromastoffe verleihen den aus dem derart modifizierten Kunststoff hergestellten Bauteilen einen angenehmen Geruch.

Grundsätzlich kommen für das Bauteil aus aromatisiertem Kunststoff beliebige im Bereich von tragbaren Spendern verwendete Kunststoffe in Frage, wie beispielsweise PBT, LDPE, HDPE und Polypropylen. Diese Kunststoffe weisen zum Zwecke der Aromatisierung vorzugsweise Additive auf, die die entsprechenden Aromen frei setzen. Diese Additive können in der DE 36 058 81 A1 vorgeschlagenen Art beim Herstellungsprozess in flüssiger Form zugegeben werden. Auch die Verwendung von Kunststoffgranulat zur Herstellung des jeweiligen Bauteils, welches bereits die entsprechenden Additive umfasst, ist möglich. Auch bei der Verwendung vom POM (Polyoxymethylen) kann die Aromatisierung zweckmäßig sein, da auch dieser Kunststoff allgemein als eher nicht wohlriechend empfunden wird. Die DE 3605881 A1 und die US 3553296 A werden durch ausdrückliche Bezugnahme im Hinblick auf ihre jeweiligen Beschreibungsteile zur Herstellung eines mit Aromastoffen hergestellten Kunststoffes zum Inhalt dieser Beschreibung gemacht.

Als Aromen kommen verschiedenste Aromen in Frage. Da die Aromen dem Zweck dienen, einen für sich genommen eher unangenehmen Geruch des Spenders und/oder der Flüssigkeit zu kompensieren bzw. zu überdecken, kommen naturgemäß als wohlriechend empfundene Aromen in Frage.

Die Aromatisierung des Kunststoffes kann auf einen oder mehrere natürliche, naturidentische und/oder synthetische Aromastoffe zurückgehen.

Insbesondere kann die Aromatisierung des Kunststoffes auf einen Aromastoffextrakt zurückgehen.

Insbesondere kann die Aromatisierung eine solche sein, die dem Aroma einer natürlichen Pflanze nachempfunden ist oder natürliche Bestandteile dieser Pflanze umfasst.

Insbesondere vorteilhaft ist es, wenn die Aromatisierung auf ein pflanzliches Öl zurückgeht, welches vorzugsweise ausgewählt ist aus der Gruppe umfassend Anisöl, Campheröl, Salbeiöl, Minzöle, insbesondere Pfefferminzöl, Citronellöl, Eukalyptusöl, Fichtennadelöl, Lavendelöl, Nelkenöl, Rosenöl, Sandelöl, Zimtöl, Citrusöle und Mischungen davon. Bevorzugte Citrusöle können aus der Gruppe umfassend Neroliöl, Petitgrainöl, Bergamottöl, Zitronenöl, Limetteöl, Orangenöl und Mischungen davon ausgewählt sein.

In einer weitergehenden Ausführungsform ist der aromatisierte Kunststoff mit einem Aromastoff versehen, welcher ausgewählt ist aus der Gruppe umfassend Kohlenwasserstoffe, Alkohole, Aldehyde, Terpene, Sesquiterpene, Ketone, Phenole, Ether, Ester, Laktone und Mischungen davon.

Beispielweise kann der aromatisierte Kunststoff mit einen Aromastoff versehen sein, welcher ausgewählt ist aus der Gruppe umfassend Ocimen, Mycren, Linalool, Geraniol, Geranylacetat, Linalylacetat, Farnesol, Farnesene, Limonen, alpha-Terpinen, Phellandren, Carvon, Menthon, Menthol, 1,8-Cineol, Bisabolol, Caryophyllene, Pinene, Camphen, Campher, Borneole und Mischungen, insbesondere Extrakte, davon.

Von besonderem Vorteil ist es, wenn ein Aroma gewählt wird, welches assoziativ auf den Zweck der jeweiligen pharmazeutischen Flüssigkeit abgestimmt ist. So wird beispielsweise Menthol häufig mit freiem Durchatmen assoziiert und eignet sich daher gut für einen Spender, mit dem eine Flüssigkeit ausgetragen wird, die dem Abschwellen der Nasenschleimhäute dient und somit das freie Durchatmen ermöglicht. Weitere als vorteilhaft empfundene Aromen sind die von Meerwasser und Seewind.

Eine vorteilhafte Anordnung des aus aromatisiertem Kunststoff hergestellten Bauteils sieht vor, dass zumindest ein Teil der Außenfläche der Nasenolive selbst aus dem aromatisierten Kunststoff besteht. Somit führt der Benutzer den aromatisierten Teil in die Nase ein oder zumindest bis in unmittelbarer Nähe seines Nasenlochs. Die Aromatisierung kann so ihren Zweck sehr gut erfüllen.

Allerdings sind auch die Kunststoffe, die an einem erfindungsgemäßen Spender verwendet werden, häufig Gegenstand von staatlichen Regulierungen, zumindest wenn sie in unmittelbarem Kontakt mit der pharmazeutischen Flüssigkeit gelangen. Um trotz möglicher Unvereinbarkeit des aromatisierten Additivs mit gesetzlichen Regularien das gewünschte Aroma vorsehen zu können, kann es von Vorteil sein, insbesondere oder ausschließlich solche Teile des Spenders aus aromatisiertem Kunststoff herzustellen, die bestimmungsgemäß nicht in Kontakt mit der auszutragenden Flüssigkeit gelangen.

Insbesondere von Vorteil ist eine Gestaltung, bei der der Spender eine Fingerauflage aufweist, die zum Zweck des Austrags der Flüssigkeit verlagerbar ist, wobei diese Fingerauflage aus dem aromatisierten Kunststoff besteht. Die Gestaltung gattungsgemäßer Spender mit austauschbaren Fingerauflagen ist aus dem Stand der Technik bereits bekannt. Entsprechend gestaltete Spender, die für eine solche austauschbare Fingerauflage ausgestaltet sind, können durch eine Fingerauflage aus aromatisiertem Kunststoff auf einfache Art und Weise erfindungsgemäß ausgestaltet werden. Die Fingerauflage ist auch deshalb ein besonders geeignetes Teil für die Anbringung des aromatisierten Kunststoffs, da sie üblicherweise unmittelbar unterhalb der Nasenolive angeordnet ist und ihr Geruch damit für den Benutzer bei der bestimmungsgemäßen Verwendung des Spenders gut erfassbar ist. Bei der aromatisierten Fingerauflage handelt es sich vorzugsweise um eine solche, die bei im übrigen bereits fertig gestelltem Spender abschließend montiert werden kann, so dass nach Abschluss der Grundmontage entschieden werden kann, ob eine derartige Fingerauflage verwendet wird und welches Aroma gewählt werden soll.

Ebenfalls zweckmäßig kann es sein, dass der erfindungsgemäße Spender eine abnehmbare Kappe aufweist, die die Austragöffnung in einem Schließzustand überdeckt und die aus dem aromatisierten Kunststoff besteht. Zwar ist die eigentliche Geruchsquelle somit während der Betätigung vom Spender entfernt. Es hat sich jedoch gezeigt, dass aufgrund der Tatsache, dass die Nasenolive im Zustand der Nichtbenutzung des Spenders von der Kappe umgeben ist, dazu führt, dass bei Benutzung das Aroma der Kappe im Bereich der Nasenolive einen zumindest kurzfristig bleibenden Geruch hinterlässt, der den gewünschten Zweck erfüllt.

Neben der genannten Gestaltung einer Nasenolive, der Fingerauflage und/oder der Kappe aus aromatisiertem Kunststoff ist auch die Verwendung anderer Gehäuseaußenwandungen des Spenders aus einem solchen Kunststoff möglich und von der Erfindung umfasst.

Insbesondere von Vorteil ist die Verwendung eines erfindungsgemäßen Spenders mit Flüssigkeiten, die selbst nicht aromatisiert sind. Die Herstellungsvorgänge zur Herstellung pharmazeutisch wirksamer aromatisierter Flüssigkeiten sind aufwendiger und teurer als die von nicht aromatisierten pharmazeutischen Flüssigkeiten. Dieser Aufwand kann durch die Aromatisierung von Teilen des Spenders, mittels dessen die Flüssigkeit ausgetragen wird, verzichtbar gemacht werden.

Weiterhin wird die Verwendung aromatisierter Kunststoffe insbesondere bei solchen Spendern als vorteilhaft angesehen, die durch eine Bestrahlung behandelt sind, insbesondere durch eine Bestrahlung mittels Gammastrahlung. Solche Spender neigen je nach Wahl des jeweiligen Kunststoffes nach der Bestrahlung zu einem unangenehmen Geruch, der sich durch die Bestrahlung der Polymerketten des Kunststoffs ergibt. Gerade bei solchen Spendern ist die erfindungsgemäße Aromatisierung von Vorteil. Es handelt sich insbesondere um solche Spender, in denen eine konservierungsmittelfreie pharmazeutische Flüssigkeit vorgesehen ist.

### Kurzbeschreibung der Zeichnungen

Weitere Vorteile und Aspekte der Erfindung ergeben sich aus den Ansprüchen sowie der nachfolgenden Beschreibung bevorzugter Ausführungsbeispiele. Dabei zeigen:
- Figur 1: einen erfindungsgemäßen Spender in einer ersten Variante und
- Figuren 2 und 3: Varianten zum Spender der Figur 1

### Detaillierte Beschreibung der Ausführungsbeispiele

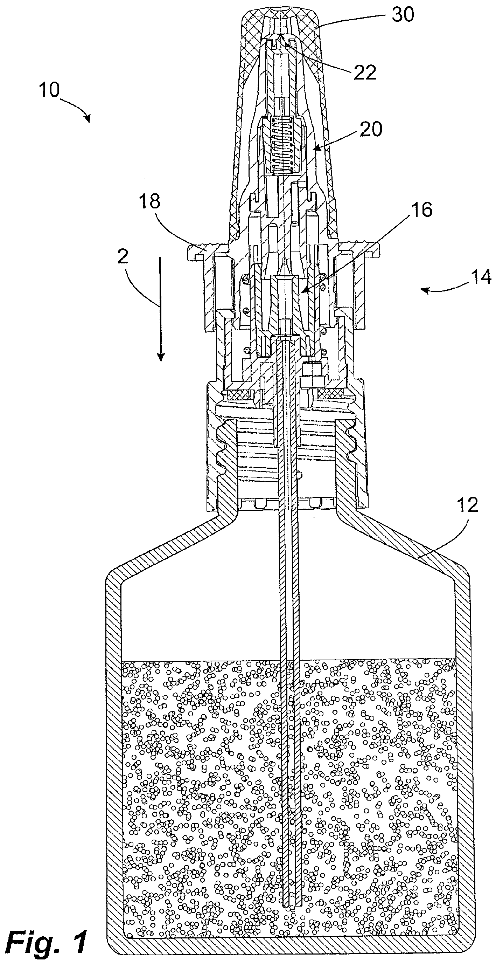

Figur 1 zeigt einen erfindungsgemäßen Spender 10. Dieser umfasst einen flaschenartigen Flüssigkeitsspeicher 12, auf den ein Austragkopf 14 aufgeschraubt ist. Der Austragkopf 14 verfügt über eine Pumpe 16, welche dadurch betätigt werden kann, dass eine Fingerauflage 18, die als Betätigungshandhabe dient, in Richtung des Pfeils 2 und auf den Flüssigkeitsspeicher 12 zu hinabgedrückt werden kann. Hierdurch kommt es zu einer Förderung von Flüssigkeit 4 aus dem Flüssigkeitsspeicher zu einer am distalen Ende einer Nasenolive 20 vorgesehenen Austragöffnung 22.

Im Zustand der Figur 1 ist die Nasenolive 20 durch eine Kappe 30 abgedeckt.

Bei dem dargestellten Spender handelt es sich um einen Spender für eine pharmazeutische Flüssigkeit 4, welche eine abschwellende Wirkung hat und bei Erkältung und Schnupfen genutzt wird. Bei dieser Flüssigkeit handelt es sich vorliegend um eine konservierungsmittelfreie Flüssigkeit. Um eine Kontamination der Flüssigkeit zu vermeiden ist der Spender 10 in seiner Gesamtheit einer Bestrahlung mit Gammastrahlen ausgesetzt worden.

Die meisten Bauteile des vorliegenden Spenders sind Kunststoffbauteile, insbesondere Kunststoffbauteile aus Polypropylen, LDPE oder HDPE. Diese Kunststoffbauteile neigen dazu, durch die genannte Bestrahlung einen unangenehmen Eigengeruch zu entwickeln. Das Einführen der Nasenolive 20 in das Nasenloch des Benutzers ist daher im Falle einer nichterfindungsgemäßen Ausgestaltung des Spenders unangenehm.

Um dem Benutzer die Verwendung zu erleichtern, ist erfindungsgemäß vorgesehen, dass zumindest ein Bauteil des Spenders 10 aus aromatisiertem Kunststoff besteht.

Im Falle der Ausgestaltung der Figur 1 ist die kreuzschraffierte Kappe 30 das Bauteil aus dem aromatisierten Kunststoff. Dieses kann durch die Aromatisierung beispielsweise mit einem Fruchtgeruch oder einem Minzgeruch versehen sein. Zwar wird die Kappe 30 bestimmungsgemäß vor dem Einführen der Nasenolive 20 in das Nasenloch abgenommen.

Da jedoch zuvor im Lagerzustand die Kappe 30 über einen längeren Zeitraum hinweg in unmittelbarer Nähe der Nasenolive 20 angeordnet war und im Idealfalle sogar mit der Nasenolive im Berührkontakt war, hat sich herausgestellt, dass der angenehme Eigengeruch der Kappe 30 zumindest kurzfristig auch im Bereich der Nasenolive 20 verbleibt, auch wenn die Kappe 30 abgenommen wurde.

Die Verwendung des erfindungsgemäßen Spenders ist somit gegenüber einem aus nicht aromatisiertem Kunststoff bestehenden Spender angenehmer.

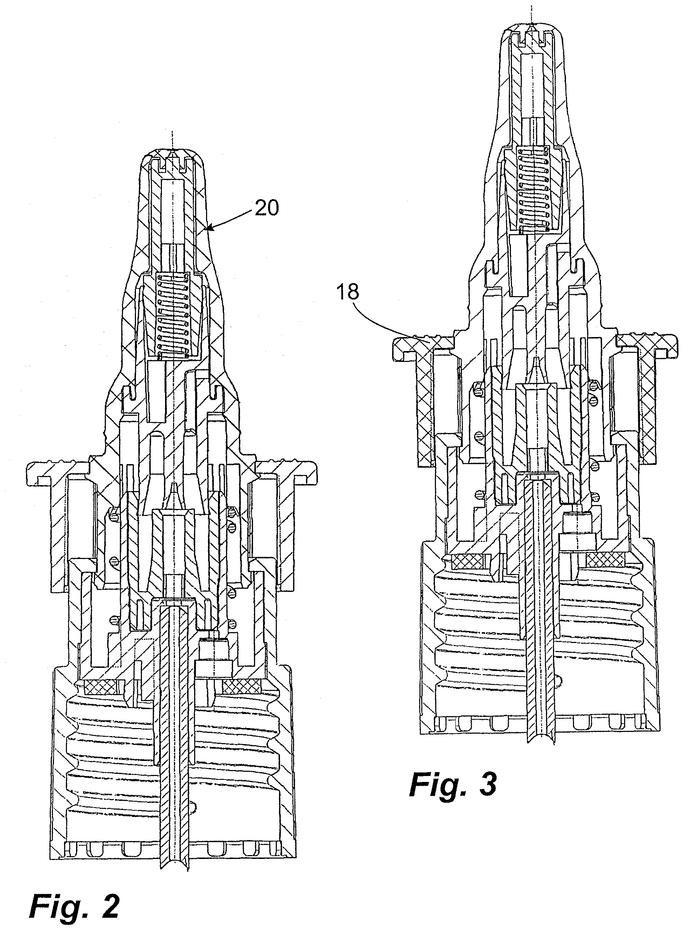

Bei der Ausgestaltung der Figur 2 ist der Spender 10 hinsichtlich seiner geometrischen Formgebung mit dem Spender der Figur 1 identisch. Allerdings ist hier, wiederum durch Kreuzschraffur verdeutlicht, die Nasenolive 20 selbst aus dem aromatisierten Kunststoff hergestellt. Hierdurch ist eine unmittelbare Nähe zwischen der Quelle des angenehmen Geruchs und dem Nasenloch des Benutzers bei Benutzung gegeben. In Hinblick auf die Wirksamkeit wird die Ausgestaltung der Figur 2 daher als die vorteilhafteste angesehen. Allerdings kann es in Hinblick auf Zulassungsregularien problematisch sein, wenn der aromatisierte Kunststoff mit der Flüssigkeit bestimmungsgemäß in Kontakt ist.

Bei einer alternativen, nicht dargestellten Variante des Spenders der Figur 2 ist die Nasenolive 20 daher zweischalig aufgebaut und weist eine nicht-aromatisierte Innenschale sowie eine aromatisierte Außenschale auf.

Bei der Ausgestaltung der Figur 3 ist die Fingerauflage 18 das Bauteil, welches aus aromatisiertem Kunststoff hergestellt ist. Dies ist deshalb von besonderem Vorteil, da die Fingerauflage 18 bestimmungsgemäß nicht in Kontakt mit der auszutragenden Flüssigkeit gelangt, bei Benutzung jedoch vergleichsweise nah am Nasenloch des Benutzers angeordnet ist und somit den angenehmen Geruch in wirksamer Weise verströmt. Hinzu kommt, dass die Fingerauflage 18 bereits bei bekannten Spendern als separate Komponente ausgebildet ist. Solche bereits bekannten Spender können in erfindungsgemäßer Weise weitergebildet werden, indem ausschließlich eine erfindungsgemäß ausgestaltete Fingerauflage Verwendung findet.

## Patentansprüche

1. Spender (10) zum Austrag einer pharmazeutischen Flüssigkeit (4) auf nasalem Wege mit
- einem Flüssigkeitsreservoir (12) und
- einer Nasenolive (20) zur Einführung in ein Nasenloch eines Benutzers,
wobei das Flüssigkeitsreservoir (12) durch einen Förderkanal mit einer Auslassöffnung (22) an der Nasenolive (20) verbunden ist, so dass durch Betätigung des Spenders ein Austragvorgang bewirkt werden kann,
**dadurch gekennzeichnet, dass**
mindestens ein Teil (30, 20, 18) des Spenders aus einem aromatisierten Kunststoff besteht, wobei die Aromatisierung mit einem Aroma aus der Gruppe umfassend Anisaroma, Campheraroma, Salbeiaroma, Minzaroma, insbesondere Pfefferminzaroma, Kamillearoma, Mentholaroma, Citronellaroma, Eukalyptusaroma, Fichtennadelaroma, Lavendelaroma, Nelkenaroma, Rosenaroma, Honigaroma und Fruchtaroma, insbesondere Beerenaroma, bevorzugt Citrusaroma, oder Mischungen davon erfolgt.

2. Spender (10) nach Anspruch 1,
**dadurch gekennzeichnet, dass**
die Aromatisierung mit einem dem Aroma einer Pflanze nachempfundenen Aroma erfolgt.

3. Spender (10) nach einem der vorstehenden Ansprüche,
**dadurch gekennzeichnet, dass**
zumindest ein Teil der Außenfläche der Nasenolive (20) selbst aus dem aromatisierten Kunststoff besteht.

4. Spender nach einem der vorstehenden Ansprüche,
**dadurch gekennzeichnet, dass**
- der Spender eine Fingerauflage (18) aufweist, die zum Zwecke des Austrags der Flüssigkeit (4) verlagerbar ist, wobei die Fingerauflage (18) aus dem aromatisierten Kunststoff besteht,
- der Spender eine Kappe (30) aufweist, die die Austragöffnung (22) überdeckt und die aus dem aromatisierten Kunststoff besteht, und/oder
- der Spender Gehäuseaußenwandungen aufweist, die aus dem aromatisierten Kunststoff bestehen.

5. Spender nach einem der vorstehenden Ansprüche,
**dadurch gekennzeichnet, dass**
ausschließlich Teile (30, 18) des Spenders (10) aus aromatisiertem Kunststoff hergestellt sind, die bestimmungsgemäß nicht in Kontakt mit der auszutragenden Flüssigkeit (4) gelangen.

6. Spender nach einem der vorstehenden Ansprüche,
**dadurch gekennzeichnet, dass**
der Flüssigkeitsspeicher (12) mit nicht-aromatisierter Flüssigkeit (4) befüllt ist.

7. Spender nach einem der vorstehenden Ansprüche,
**dadurch gekennzeichnet, dass**
der Spender (10) zumindest teilweise durch Bestrahlung behandelt ist, insbesondere mittels Gamma-Strahlung.

## Claims

1. Dispenser (10) for discharging a pharmaceutical liquid (4) by the nasal route, having
- a liquid reservoir (12) and
- a nasal olive (20) for insertion into a nostril of a user,
wherein the liquid reservoir (12) is connected by a feed channel to an outlet opening (22) in the nasal olive (20) so that, by actuating the dispenser, a discharge operation can be effected,
**characterized in that**
at least one part (30, 20, 18) of the dispenser is made of an aromatized plastics material, wherein the aromatization is carried out with an aroma from the group comprising aniseed aroma, camphor aroma, sage aroma, mint aroma, in particular peppermint aroma, chamomile aroma, menthol aroma, citronella aroma, eucalyptus aroma, pine-needle aroma, lavender aroma, carnation aroma, rose aroma, honey aroma and fruit aroma, in particular berry aroma, preferably citrus aroma, or mixtures thereof.

2. Dispenser (10) according to Claim 1,
**characterized in that**
the aromatization is carried out with an aroma based on the aroma of a plant.

3. Dispenser (10) according to one of the preceding claims,
**characterized in that**
at least part of the outer surface of the nasal olive (20) itself is made of the aromatized plastics material.

4. Dispenser according to one of the preceding claims,
**characterized in that**
- the dispenser has a finger rest (18) which is displaceable for the purpose of discharging the liquid (4), wherein the finger rest (18) is made of the aromatized plastics material,
- the dispenser has a cap (30) which covers the discharge opening (22) and which is made of the aromatized plastics material, and/or
- the dispenser has housing outside walls which are made of the aromatized plastics material.

5. Dispenser according to one of the preceding claims,
**characterized in that**
only parts (30, 18) of the dispenser (10) that do not normally come into contact with the liquid (4) to be discharged are made of aromatized plastics material.

6. Dispenser according to one of the preceding claims,
**characterized in that**
the liquid reservoir (12) is filled with non-aromatized liquid (4).

7. Dispenser according to one of the preceding claims,
**characterized in that**
at least part of the dispenser (10) has been treated by irradiation, in particular by means of gamma radiation.

## Revendications

1. Distributeur (10) pour distribuer un liquide pharmaceutique (4) par voie nasale, présentant
- un réservoir (12) à liquide et
- un embout nasal (20) destiné à être introduit dans une narine d'un utilisateur,
de réservoir (12) à liquide étant relié par un canal de transport à une ouverture (22) de distribution au niveau de l'embout nasal (20) de manière telle que par l'actionnement du distributeur, un processus de distribution peut être provoqué, **caractérisé en ce qu'**au moins une partie (30, 20, 18) du distributeur est constituée par un matériau synthétique aromatisé, l'aromatisation ayant lieu avec un arôme du groupe comprenant l'arôme d'anis, l'arôme de camphre, l'arôme de sauge, l'arôme de menthe, en particulier l'arôme de menthe poivrée, l'arôme de camomille, l'arôme de menthol, l'arôme de citronnelle, l'arôme d'eucalyptus, l'arôme d'aiguilles de pin, l'arôme de lavande, l'arôme de girofle, l'arôme de rose, l'arôme de miel et l'arôme de fruits, en particulier l'arôme de baies, de préférence l'arôme d'agrumes ou des mélanges de ceux-ci.

2. Distributeur (10) selon la revendication 1, **caractérisé en ce que** l'aromatisation a lieu avec un arôme inspiré par l'arôme d'une plante.

3. Distributeur (10) selon l'une quelconque des revendications précédentes, **caractérisé en ce qu'**au moins une partie de la surface externe de l'embout nasal (20) lui-même est constituée par le matériau synthétique aromatisé.

4. Distributeur selon l'une quelconque des revendications précédentes, **caractérisé en ce que**
- le distributeur présente un emplacement (18) pour les doigts, qui peut être déplacé en vue de la distribution du liquide (4), l'emplacement (18) pour les doigts étant constitué par le matériau synthétique aromatisé,
- le distributeur présente un capuchon (30), qui recouvre l'ouverture de distribution (22) et qui est constitué par le matériau synthétique aromatisé, et/ou
- le distributeur présente des parois externes de boîtier qui sont constituées par le matériau synthétique aromatisé.

5. Distributeur selon l'une quelconque des revendications précédentes, **caractérisé en ce que** seules les pièces (30, 18) du distributeur (10) qui, lors de l'utilisation normale, n'entrent pas en contact avec le liquide (4) à distribuer sont fabriquées en matériau synthétique aromatisé.

6. Distributeur selon l'une quelconque des revendications précédentes, **caractérisé en ce que** le distributeur (12) de liquide est rempli par un liquide (4) non aromatisé.

7. Distributeur selon l'une quelconque des revendications précédentes, **caractérisé en ce que** le distributeur (10) est au moins partiellement traité par irradiation, en particulier par un rayonnement gamma.
